# EUROPÄISCHE PATENTSCHRIFT

(11) **EP 3 093 011 B1**
(45) Veröffentlichungstag und Bekanntmachung des Hinweises auf die Patenterteilung: **24.03.2021**
(21) Anmeldenummer: 16001371.0
(22) Anmeldetag: 17.11.2011
(51) Int. Cl.: A61K 9/16, A61K 9/20, C07C 29/76, C07C 31/26, C07C 29/78

(54) **VERFAHREN ZUR HERSTELLUNG VON DIREKT TABLETTIERBAREM DELTA-MANNIT**
METHOD FOR THE PREPARATION OF DIRECTLY TABLETTABLE MANNITOL
PROCEDE DE FABRICATION DE MANNITOL A MONTER DIRECTEMENT SUR TABLETTE

(30) Priorität: 17.12.2010 EP 10015770
(43) Veröffentlichungstag der Anmeldung: 16.11.2016
(62) Teilanmeldung aus: 11784604.8
(73) Patentinhaber: Merck Patent GmbH, 64293 Darmstadt (DE)
(72) Erfinder: Erdmann, Martin, 64521 Gross-Gerau (DE); Hamm, Walter, 64331 Weiterstadt (DE); Schwarz, Eugen, 83512 Wasserburg (DE)

(56) Entgegenhaltungen:
- EP-A1- 1 319 644
- EP-A1- 1 967 211
- WO-A1-2004/020648
- HULSE WENDY L ET AL: "The characterization and comparison of spray-dried mannitol samples.", DRUG DEVELOPMENT AND INDUSTRIAL PHARMACY JUN 2009 LNKD- PUBMED:19514986, Bd. 35, Nr. 6, Juni 2009 (2009-06), Seiten 712-718, XP002678343, ISSN: 1520-5762
- FRANK R. FRONCZEK ET AL: "-mannitol at 100 K", ACTA CRYSTALLOGRAPHICA SECTION C CRYSTAL STRUCTURE COMMUNICATIONS, Bd. 59, Nr. 10, 23. September 2003 (2003-09-23), Seiten o567-o570, XP055123554, ISSN: 0108-2701, DOI: 10.1107/S0108270103018961

## Beschreibung

Die vorliegende Erfindung betrifft ein Verfahren zur Herstellung von direkt tablettierbarem Mannit mit einem Gehalt an δ-Modifikation von mehr als 90 %, insbesondere von mehr als 95 %.

Bei der Herstellung von Tabletten kann als Trägermaterial für einen Wirkstoff D-Mannit eingesetzt werden. Dazu wird üblicherweise D-Mannit in mehreren Verfahrensschritten in ein Granulat übergeführt, um es für die Tablettierpressen handhabbar zu machen und gleichzeitig eine Wirkstoffeinbindung zu ermöglichen. Zwischenkontrollen ermöglichen hierbei eine kontrollierte Prozessführung.

Aus US 3,145,146 A ist ein Sprühtrocknungsverfahren bekannt, wodurch Mannit in Form feiner Partikel mit einem durchschnittlichen Durchmesser von 5 bis 150 µm erhalten wird. Eine Mannit-Lösung wird durch Versprühen in einen Heißgasstrom sprühgetrocknet. Die erhaltenen Partikel werden durch geeignete Maßnahmen abgetrennt

Zur Herstellung von granulatförmigem D-Mannit ist auch die Granulierung in einem Wirbelbett bekannt, worin der Prozeßluftstrom einen speziell geformten Anströmboden durchströmt und so eine Wirbelschicht aus festem Startmaterial erzeugt. Die Sprühflüssigkeit gelangt durch ein Düsensystem feinverteilt in den Wirbelraum. Die wirbelnden Partikel werden benetzt, die Oberfläche angelöst, und die Partikel haften zusammen und trocknen in dem Luftstrom, der sie zum Ausgang des Wirbelbettes trägt. Am Ende des Wirbelbettes wird kontinuierlich Feststoff entnommen. Gleichzeitig wird am Eingang eine geringere Menge des Bettes durch zurückgeführten Feststoff gebildet, auf den Sprühflüssigkeit fein verteilt wird. Ein Filtersystem bewirkt, daß kein Staub das Wirbelbett verläßt und nur am Ausgang Granulatpartikel entnommen werden, die eine Mindestgröße aufweisen. Auch in einem solchen Wirbelbett bilden sich Feststoffpartikel, die mehr oder weniger statistisch geformt sind. Entsprechende Anlagen werden durch verschiedene Hersteller vertrieben.

Üblicherweise schließt sich an die Herstellung eines granulatförmigen Mannits eine Verfahrensstufe an, durch die ein Granulat mit einer gleichförmigen Partikelgrößenverteilung erhalten wird. Diese Verfahrensstufe kann sowohl ein Mahlen als auch ein Sieben (Klassieren) des Granulates einschließen. Im Fall der Verwendung von Mannit als Trägermaterial für pharmazeutische Wirkstoffe stellt für den Fachmann jeder zusätzliche Verfahrensschritt in der Herstellung eine mögliche Gefahr dar, unerwünschte Verunreinigungen in das Produkt einzutragen.

Aus der Literatur ist, bekannt, dass D-Mannit in polymorphen Kristallformen vorliegen kann, und zwar in der α-, β- und δ-Form. Die hier benutzten Definitionen und Charakterisierungen entsprechen denen in : Walter Levy, L.; Acad. Sc. Paris, t. 267 Series C, 1779, (1968) gegebenen Klassifikationen der polymorphen Formen durch Röntgenstrukturanalyse (X-ray diffraction pattern). Die β-Form ist die stabilste Form, obwohl Umwandlungen in die anderen Formen in Abhängigkeit von der Lagerzeit und den Umgebungsbedingungen möglich sind. Für kommerzielle Anwendungen wäre es daher wünschenswert, Mannit aufgrund seiner Stabilität direkt bei der Herstellung in der β-Form zu erhalten, da sich die Produkteigenschaften in diesem Fall am wenigsten durch Lagerung ändern. Um in der Herstellung von pharmazeutischen Formulierungen einen Wirkstoff gleichmäßig verteilt zu halten, insbesondere in pulverförmigen Formulierungen, ist es wünschenswert, den Wirkstoff in dem Trägermaterial (Excipient) fest einzubinden.

Es ist einerseits für die Tablettiereigenschaften pulverförmigen D-Mannits von Bedeutung, in welcher polymorphen Form es vorliegt und andererseits in welcher Weise die Kornstruktur der einzelnen Partikel aufgebaut ist.

Aus WO2004/020648 A1 ist ein Verfahren zur Herstellung von direkt tablettierbarem Mannit in der α-Kristallform beschrieben, wobei zu seiner Herstellung D-Mannit mit einer Reinheit von > 98 Gew.% eingesetzt wird. Die Herstellung erfolgt in einer Sprühtrocknungsanlage mit Wirbelbett, in der ein Teil des hergestellten Produkts aus der Produktaustragszone zurückgeführt wird, derart dass Mannitlösung, Sprühgas, pulverförmiges α-Mannit und Heißgas in einer Sprühtrocknungseinheit zusammengeführt werden, und wobei die wässrige D-Mannitlösung mindestens 45-%ig ist und mit einer Temperatur im Bereich von 60 bis 100 °C versprüht wird.

Aus EP 1319644 B2 wiederum ist ein Verfahren bekannt zur Herstellung von direkt tablettierbarem D-Mannit in β-Kristallform, β-Mannit. Das Verfahren zur Herstellung des β-Mannits erfolgt in derselben Anlage wie die vorab beschriebene Herstellung von α-Mannit, wobei jedoch im Unterschied dazu für die Bildung der β-Mannit-Kristallform induziert wird, indem aus der Produktaustragszone der Anlage Pulver mit Teilchengrößen kleiner 20 µm rückgeführt wird, und durch Einstellung des Gleichgewichts in der Anlage ein Produkt erhalten wird mit einem Anteil an β-Fraktion von mehr als 90%, wobei die Temperatur der zugeführten Luft im Bereich von 45 - 120 °C liegt und die Ablufttemperatur im Bereich zwischen 30 bis 50 °C liegt.

Nachteile dieses Verfahrens sind, dass sich die Umwandlung als zusätzlicher Verfahrensschritt an die eigentliche Pulverherstellung anschließt, die Trocknung mindestens 8 Stunden erfordert, wobei der Anlage kontinuierlich Wärmeenergie zugeführt werden muss, und dass bisher keine gezielte Steuerungsmöglichkeit bekannt ist, durch die Mannit mit einem sehr hohen Anteil in δ-Form erhalten wird.

### Aufgabe

Aufgabe der vorliegenden Erfindung ist es daher, ein Verfahren zur Verfügung zu stellen, das in einfacher Weise durchführbar ist und durch das in einem Arbeitsschritt ein granulatförmiges, direkt tablettierbares Mannit in der δ-Modifikation herstellbar ist.

### Beschreibung der Erfindung

Die Lösung der Aufgabe erfolgt durch ein Verfahren zur Herstellung von direkt tablettierbarem Mannit mit einem Gehalt an δ-Modifikation von mehr als 90 %, dadurch gekennzeichnet, dass
a) ein Wirbelbett aus sehr feinteiligem β-Mannitpulver hergestellt wird, indem DC-β-Mannit als Bett im Prozessor vorgelegt wird und
b) in einem ersten Schritt als Edukt eine wässrige D-Mannit-Lösung, Sprühgas, das β-Mannitpulver und Heißluft zusammengeführt werden,
   und
c) das entstehende granulatförmige Produkt in das Wirbel- oder Fließbett fällt, aufgenommen, fluidisiert wird, und gegebenenfalls in einem oder mehreren Granulierungsschritt(en) mit weiterem flüssigen Medium besprüht, getrocknet, und im Fließbett in Richtung der Dosieranlage weitertransportiert wird,
   und anschließend
d) eine Teilmenge ungemahlen als pulver- und/oder granulatförmiges Material in den Prozess zurückgeführt wird,
   wobei
e) im Anfahrprozess die Zuluft des Mahlventilators (E) staubfrei ist, in der Anlage die Klappe (H) geschlossen ist und die Klappe (J) geöffnet ist und die Luft aus der Abluft des Prozessors nach den Filtern (G) entnommen wird
   und
f) im Prozessor eine Temperatur von max. ca. 35 - 45 °C sowie ein Sprühdruck von max. 2,5 - 3,0 bar eingestellt werden,
   wobei die D-Mannit-Lösung hergestellt ist aus D-Mannit mit einem Mannitgehalt von >98 Gew.%, und die Rückführung ungemahlenen Pulvers in einer Menge von ca. der 3-fachen Menge erfolgt, bezogen auf die aus dem Prozess entnommene Produktmenge, und das rückgeführte Pulver in der Anlage den Weg 9B/10B nimmt, wobei der Weg 9A/10A geschlossen ist, wodurch die Bildung von δ-Mannit einsetzt und die Konzentration an δ-Mannit unter den eingestellten Bedingungen kontinuierlich zunimmt,
   und
g) wenn der Gehalt an δ-Mannit im gebildeten Produkt auf etwa 60 - 70 Gew.-% gestiegen ist, die Rückführung von staubhaltiger Zuluft umgestellt wird, indem in der Anlage die Klappe (H) geöffnet und die Klappe (J) geschlossen werden, wodurch der Gehalt an δ-Mannit auf über 90 Gew.-% im Produkt gesteigert wird.

Wie gesagt, kann eine Teilmenge des entstehenden granulatförmigen Produkts in den Prozess zurückgeführt werden. Diese Rückführung von gebildetem Produkt ist besonders vorteilhaft, wenn darin mehr als 90 % in δ-Form vorliegt. In dem Verfahren kann zu Beginn Mannit zugeführt werden mit einem Gehalt an δ-Modifikation von mehr als 90 %, welches aus einer konventionellen Herstellung des δ-Mannits durch Kristallisation stammen kann, wobei jedoch auf eine geringe Partikelgröße zu achten ist, die durch Mahlen und Sieben eingehalten werden kann. Erst später nach Erreichen des Gleichgewichts in der Anlage, wobei im Verfahren kontinuierlich Mannit mit einem Gehalt an δ-Modifikation von mehr als 90 % erhalten wird, spielt die Partikelgröße nur noch eine untergeordnete Rolle.

Zur Herstellung der Mannit-Lösung wird D-Mannit mit einer Reinheit von >98 % verwendet.

Überraschenderweise lässt sich das Gleichgewicht hin zur Bildung von δ-Mannit verschieben, indem als Staubanteil anfallendes δ-Mannits aus der Produktaustragszone des Prozessors in den Schritt a) der Sprühtrocknung rückgeführt wird. Besonders vorteilhaft gestaltet sich das Verfahren, indem δ-Mannit-Staub mit einer durchschnittlichen Partikelgröße kleiner 20 µm, insbesondere mit durchschnittlicher Partikelgröße im Bereich von etwa 1 bis 20 µm, vorzugsweise im Bereich von 3 bis 15 µm, zurückgeführt wird.

Die Rückführung von "staubförmigem" δ-Mannit, das als Produktausschleusung in der Dosieranlage in der Leitung (9A) anfällt, erfolgt durch Steuerung der Drehzahl der Zellradschleuse 10A über den Ventilator (E) in die Sprühtrocknung des Verfahrensschritts a). Dieses heißt, dass das Granulat der Produktrückführung im Mahlventilator zu Pulver gemahlen wird.

Nach der Einstellung des Gleichgewichts ist es ohne weiteres möglich, pulverförmiges δ-Mannit mit einer durchschnittlichen Partikelgröße kleiner als 75 µm rückzuführen, insbesondere aber auch pulverförmiges unvermahlenes δ-Mannit (9B, 10B).

Da in dem erfindungsgemäßen Verfahren, bzw. Sprühtrocknungsverfahren aus einer Lösung oder Suspension ein Feststoff mit speziellen Eigenschaften generiert wird, soll hier unterschieden werden zwischen den verschiedenen Feststoffformen Granulat, Pulver und Staub.

Wenn hier von einem Granulat gesprochen wird, handelt es sich um einen körnigen, leicht schüttbaren Feststoff mit einer spezifischen Struktur, wie es das erfindungsgemäß hergestellte δ-Mannits aufweist. Ein Granulat besteht also aus festen und trockenen Körnern, wobei jedes Korn wiederum ein Agglomerat aus Partikeln mit genügender Festigkeit darstellt, um verschiedene Handhabungen zuzulassen.

Wenn dagegen von Pulver gesprochen wird, handelt es sich um Feststoffe aus festen, losen, trockenen und mehr oder weniger feinen Teilchen, vorzugsweise sehr feinen Teilchen, die durch Mahlen des erfindungsgemäß hergestellten Granulats mit dem Mahlventilator erhalten werden. Diese Teilchen weisen keine spezifische Struktur auf, weil sie beim Mahlen zerstört worden ist.

Staub wiederum besteht aus sehr feinen Partikeln, die sich in Luft nicht oder nur schwer absetzen (< 10µm), hier erfindungsgemäß anfallender feinster Feststoff aus der Gasphase (zwischen Fließbett und Filter) des Prozessors. Die Partikel sind so fein, dass sie nicht sedimentieren können.

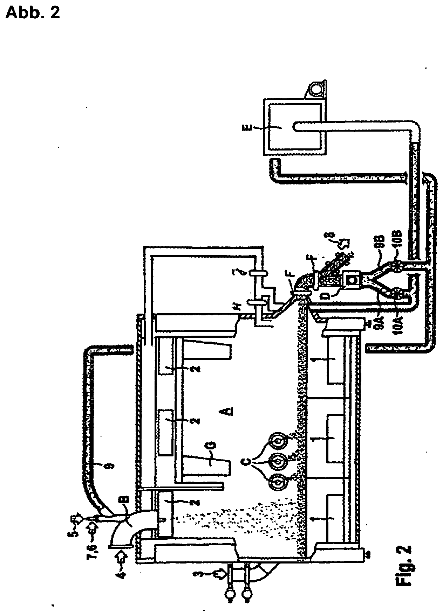

Die besondere Ausgestaltung der verwendeten Anlage ermöglicht es, dass das zurückgeführte granulatförmige Material vor der Rückführung durch Vermahlen im Ventilator [(E), Abb. 2] der gleichzeitig als Förderorgan für die Pulverrückführung dient, zerkleinert wird.

Durch Regelung der Drehzahlen der Zellradschleusen 10A und 10B (Fig. 2) der verwendeten Anlage und durch Vermahlen des anfallenden grobkörnigen (Überkorn) Produkts im Ventilator [(E), Abb. 2] auf Partikelgrößen kleiner 75 µm vor der Rückführung in die Sprühtrocknung erfolgt die alleinige Bildung von δ-Mannit.

Zur Durchführung des Verfahrens wird eine wässrige 40 - 50%-ge D-Mannit-Lösung (bezogen auf das Gewicht der Gesamtlösung) als Edukt eingesetzt, die mit einer Temperatur im Bereich von 60 bis 100 °C versprüht wird.

Sowohl als Sprühgas als auch als Träger- und Heizgas kann Luft oder ein Inertgas ausgewählt aus der Gruppe N₂ und CO₂ verwendet werden. Das Gas wird im erfindungsgemäßen Verfahren vorzugsweise im Kreislauf geführt und das im Kreislauf geführte Gas durch Filter von Partikeln befreit, im Kondensator getrocknet und erneut den Sprühdüsen zugeführt bzw. aufgeheizt und in das Fließbett eingeführt.

Vorzugsweise wird das im Kreis geführte Gas mit Hilfe von Dynamikfiltern von Partikeln befreit.

In einer besonderen Ausführungsform des Verfahrens weisen die verwendeten flüssigen Medien an verschiedenen Stellen der Anlage unterschiedliche Zusammensetzungen auf.

Durch Variation der Verfahrensparameter, Sprühdruck, Sprühmenge, Mannitkonzentration, zurückgeführte Pulvermenge, Heißluftstrom und Temperatur der Heißluft lassen sich im erfindungsgemäßen Verfahren gezielt Partikelgrößen zwischen 50 bis 1000 µm herstellen.

Zu diesem Zweck wird erfindungsgemäß die der Anlage zugeführte Luft auf eine Temperatur im Bereich von 45 - 110 °C vorgewärmt und die zugeführte Zuluftmenge im Bereich von 1000 - 2000 m³/m² pro Stunde geregelt, wodurch sich die Ablufttemperatur im Bereich von 30 - 50 °C einstellt. Gleichzeitig wird der Sprühdruck der Zweistoffdüsen im Bereich von 2 - 4 bar eingestellt so dass etwa 1,5 bis 3 m³/(h kg Lösung) Heißgas der Zweistoffdüse zugeführt werden, wobei die Temperatur des Heißgases im Bereich von etwa 80 bis 110 °C eingestellt wird. Gute Verfahrensergebnisse werden erhalten, wenn die Pulver- und Granulatrückführung so geregelt wird, dass eine Rückführung in einer Menge im Bereich von 0,5 - 2,0 kg Feststoff/(h kg Lösung) erfolgt.

Eine besonders gleichförmige Bildung von granulatförmigem Pulver mit einem δ-Mannitgehalt von >95% erfolgt durch Einstellung der Parameter, Sprühdruck, Flüssigkeitsmenge, Mannitkonzentration, zurückgeführte Pulver- und Granulatmenge, Heißluftstrom und Temperatur der Heißluft, wodurch die im Wirbel- oder Fließbett befindliche Pulvermenge auf eine Bettmenge im Bereich von 50 - 150 kg/m² Bett eingestellt wird.

Durch entsprechende Versuche wurde somit ein Verfahren zur Herstellung von reinem δ-(D)-Mannit entwickelt, durch das direkt tablettierbares Mannit (DC-Mannit) mit geeigneter homogener Partikelgrößenverteilung herstellbar ist. Zur Durchführung des Verfahrens wird Mannit mit einer Reinheit von über 98% eingesetzt, wobei der Rest Sorbit und andere Restzucker sein können. Es wird eine wässrige Lösung mit einem Mannitgehalt von ca. 40 - 50 Gew.-% hergestellt. Diese Lösung wird mit einer Zulufttemperatur von ca. 60 - 95 °C in einer Sprühtrocknungsanlage versprüht und getrocknet.

Zur Durchführung des Verfahrens wird eine leicht veränderte Anlage eingesetzt wie in DE 1 99 27 537 beschrieben. Mit der in dieser Patentanmeldung beschriebenen Anlage ist es an sich möglich, die Eigenschaften von sprühgetrockneten und granulierten Produkten nach Wunsch hinsichtlich Korngröße, Korngrößenverteilung, Feuchte und Tablettierfähigkeit zu variieren. Die durchgeführten Veränderungen der Anlage ermöglichen jedoch eine zusätzliche Feinregulierung durch die Pulver- und Granulatrückführung.

Insbesondere erfolgt die Durchführung des Verfahrens in einer Sprühtrocknungsanlage, welche
a) eine Sprühtrocknungseinheit (B)
b) ein Fließbett (A)
c) eine oder mehrere zusätzliche Sprüh- oder Zerstäubungsdüsen für flüssige Medien (C)
e) einem Dosiergerät (D) zur Produktrückführung und
f) einer Pulver- und Granulatrückführung (9) mit Ventilator (E) aufweist,
wobei die für die Pulver- und Granulatrückführung vorgesehenen Leitungen (9A) und (9B) mit Zellradschleusen (10A, 10B) versehen sind, und das nicht in die Pulverdosierung eintretende Produkt (8) durch Klassierung in eine staubförmige und eine grobkörnige Fraktion getrennt werden kann. Dem Ventilator (E) kann je nach Bedarf und Stadium der Gleichgewichtseinstellung des Prozesses entweder staubfreie Zuluft über die Klappe (J) oder staubhaltige Zuluft über die Klappe (H) zugeführt werden (siehe Abb. 2).

In der Sprühtrocknungseinheit (B) der erfindungsgemäß verwendeten Anlage werden flüssiges Medium (5), Sprühgas (6), pulverförmiges Material (9) und Heißgas (4) zusammengeführt.

Eine besondere Ausführungsform besteht darin, daß sich eine Sprühtrocknungseinheit (B) senkrecht über einem anschließenden Wirbelbett in einem Sprühturm befindet.

In einer speziellen Ausführungsform kann die Sprühtrocknungseinheit (B) der Anlage ein Sprühsystem enthalten, das aus einer mit Heißwasser beheizten Zweistoff-Sprühdüse mit koaxial angeordneter Pulver und Granulatrückführung und Heißgasumströmung besteht.

In der verwendeten Anlage können im Fließbett auch örtlich variabel eine oder mehrere zusätzliche Sprüh- oder Zerstäubungsdüsen für flüssige Medien (C) angebracht sein. An das Fließbett schließt sich eine durch eine Schleusenklappe (F) abgetrennte Pulverdosieranlage (D) an, welche durch einen Produktüberlauf (8) gespeist wird. Eine Teilmenge des gebildeten Produkts kann über eine Flugförderung, in der ein Ventilator (E) als Förderorgan dient, nach Zerkleinerung (9A,10A) oder auch unzerkleinert (9B, 10B), wenn das Gleichgewicht eingestellt ist, in die Sprühtrocknungseinheit (B) zurückgeführt werden. Der als Förderorgan wirkende Ventilator (E) kann gleichzeitig als Zerkleinerungseinheit für rückgeführtes Pulver dienen.

Die Durchführung des Verfahrens zur Herstellung von sprühgetrocknetem granulatförmigen δ-(D)-Mannit erfolgt, indem
a) in einem ersten Schritt ein flüssiges Medium, Sprühgas, pulverförmiges Material und Heißluft zusammengeführt werden,
b) das entstehende granulatförmige Produkt in ein Fließbett fällt, aufgenommen, fluidisiert und weitertransportiert wird und gegebenenfalls
c) in einem oder mehreren Granulierungsschritt(en) mit weiterem flüssigen Medium besprüht, getrocknet
d) und im Fließbett in Richtung der Dosieranlage gefördert wird, von welcher aus
e) eine Teilmenge ungemahlen als pulver- und/oder granulatförmiges Material in den Prozeß zurückgeführt wird.

Bei dem flüssigen Medium handelt es sich um eine wässrige Lösung. Es kann aber auch eine Lösung eingesetzt werden, in der Lösungsmittelgemisch eingesetzt worden ist, das zu einem bestimmten Anteil aus Wasser besteht.

Als Sprüh-, Träger- und Heizgas kann Luft oder ein Inertgas ausgewählt aus der Gruppe N₂ und CO₂ verwendet werden. Das Gas kann erfindungsgemäß im Kreislauf geführt werden, wobei es durch Filter oder speziell mit Hilfe von Dynamikfiltern von Partikeln befreit wird, im Kondensator getrocknet und erneut den Sprühdüsen zugeführt bzw. aufgeheizt und in das Fließbett eingeführt wird.

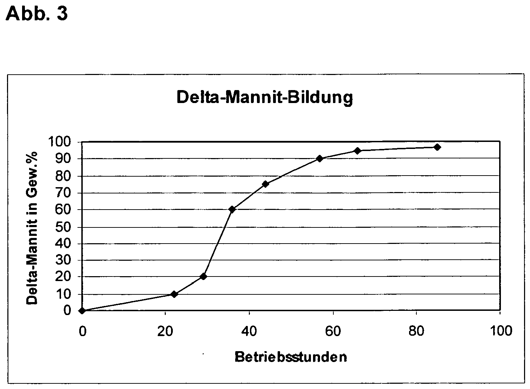

Zur Durchführung des Verfahrens zur Herstellung von granulatförmigem δ-(D)-Mannit im Wirbelbett stellt man ein solches Wirbelbett aus sehr feinteiligem β-Mannitpulver her. Dazu muss DC-β-Mannit als Bett im Prozessor vorgelegt werden. Das DC-β-Mannit hat sehr ähnliche mechanische Eigenschaften wie das herzustellende DC- δ-Mannit, der Anfahrprozess ist dadurch stabiler als ein Start mit kristallinem δ-Mannit (siehe Fig. 3). Um das gewünschte δ-Mannit zu erhalten, müssen im Prozess folgende Parameter eingestellt werden:
- Temperatur im Prozessor:: max. ca. 35 - 45 °C
- Sprühdruck:: max. 2,5 - 3,0 bar
- Pulverrückführung:: ca. 3-fache Menge, bezogen auf die aus dem Prozess entnommenen Produktmenge Das rückgeführte Pulver wird nicht gemahlen! Das Pulver nimmt den Weg 9B/10B (Der Weg über 9A/10A ist geschlossen!)
- Mannitgehalt :: >98 Gew.%

An dieser Stelle ist es entscheidend, dass die Zuluft des Mahlventilators staubfrei ist. In diesem Fall ist in der Anlage die Klappe (H) geschlossen und die Klappe (J) ist geöffnet. Die Luft wird aus der Abluft des Prozessors nach den Filtern (G) entnommen. Bei dieser Einstellung setzt die Bildung des δ-Mannits ein und nimmt den Verlauf wie in Abb. 3 gezeigt. Zu Beginn des kontinuierlich geführten Prozesses erfolgt die Bildung von δ-Mannit in geringen Mengen. Unter den eingestellten Bedingungen, wie oben beschrieben, nimmt die Konzentration kontinuierlich zu, bis sie nach einer Betriebszeit von etwa 70 Stunden etwa 95 Gew.-% beträgt. Im Anschluss daran steigt die Konzentration an δ-Mannit nur noch geringfügig und kann einen Wert von mehr als 98 Gew.-% betragen. Wenn der Gehalt an δ-Mannit gegen die Betriebsdauer aufgetragen wird, ergibt sich eine S-Kurve, wie in Abb. 3 zu sehen ist.

In der Durchführung des Verfahrens hat sich gezeigt, dass der Prozess umgestellt werden muss, wenn der Gehalt an δ-Mannit im gebildeten Produkt auf etwa 60-70 Gew.-% gestiegen ist. Ab diesem Zeitpunkt muss der Prozess auf die Rückführung von staubhaltiger Zuluft umgestellt werden, wie in Fig. 2 dargestellt ist. In diesem Fall ist in der Anlage die Klappe (H) offen und die Klappe (J) ist geschlossen. Nur unter diesen Bedingungen lässt sich der Gehalt an δ-Mannit auf über 90 Gew.-% im Produkt steigern.

Um in dem erfindungsgemäßen kontinuierlichen Verfahren stabil δ-Mannit mit einer derart hohen Konzentration herstellen zu können, muss feinststaubhaltige (Mannit-Feinstaub) Luft als Zuluft für den Mahlventilator eingesetzt werden und die oben beschriebenen Parameter eingehalten werden. Bei Über- oder Unterschreiten dieser Werte rutscht der Prozess aus dem Gleichgewicht, und es bildet sich bevorzugt wieder β-Mannit. Weiterhin muss das gebildete Produkt bis auf eine Restfeuchte kleiner als 1%, insbesondere kleiner 0,3%, getrocknet werden, um ein stabiles, lagerfähiges Produkt zu erhalten, da das Wasser das δ-Mannit in die β-Modifikation umwandelt.

Zum Anfahren des Prozesses wird die Anlage zu Beginn mit pulverförmigem Startermaterial über den Einfüllstutzen (3) beschickt. Über die Kammern (1) wird im Sprühtrocknungsraum ein Luftstrom erzeugt. Das eingeführte Startermaterial wird durch diesen Luftstrom fluidisiert und bewegt sich in die Richtung der Austragsklappen (F). Der Produktstrom erhält diese Bewegungsrichtung bei Erzeugung des Luftstroms durch eine entsprechende Perforation des Conidurbodens. Das fluidisierte Produkt läßt sich durch einfaches öffnen der Schleusenklappen (F) austragen. An dieser Stelle der Anlage sind Vorrichtungen geschaffen, die es ermöglichen, das Produkt in eine Dosieranlage und eine Flugförderung zur Sprühtrocknungseinheit zurück zu führen. Am Austrag über der Dosieranlage befindet sich ein Überlauf (8) für das Fertigprodukt. Der Ventilator (E) der Sprühtrocknungseinheit dient als Fördermittel sowohl für die Produktrückführung als auch als Zerkleinerungseinheit für zurückzuführendes Pulvermaterial. Die Zuluft erhält der Ventilator (E) entweder staubbeladen über die Klappe (H) oder staubfrei über die Klappe (J). Zurückgeführtes Pulver- und Granulatmaterial der Rückführungsleitung (9A, 9B) wird durch die besondere Ausgestaltung der Sprühtrocknungsdüse mit den entsprechenden Medien Flüssigkeit (5), Sprühluft (6) und Heißluft (4) zusammengeführt. Das entsprechende Pulver bzw. Granulat wird vom Fließbett aufgenommen und, wie bereits oben beschrieben, weitertransportiert. Beim Passieren der Granulationsdüsen (C) kann weiteres Medium, welches eine andere Zusammensetzung haben kann als das in die Sprühdüse mit Pulverrückführung eingebrachte, auf die gebildeten Partikel gesprüht. Auf diese Weise können ein weiteres Granulieren und eine erneute Einstellung der Korngrößenverteilung erfolgen. Das Produkt aus den Kammern (1) wird durch Luft, welche über die Conidurböden eingeschleust wird, auf die gewünschte Endfeuchte getrocknet. Durch in die Anlage integrierte Dynamikfilter (G) wird ein Austrag von Pulverpartikeln in die Umgebung verhindert.

Statt der Granulationsdüsen (C), wie in Abb. 2 dargestellt, können an der entsprechenden Stelle der Anlage ein oder mehrere Sprühdüsen oder Sprühtrocknungsdüsen oder auch nur eine, zwei oder mehr als drei Granulationsdüsen angebracht sein. Diese zusätzlichen Düsen können sich direkt am Anfang des Fließbettes oder weiter nach hinten verschoben befinden. Die Wahl des Ortes, an dem das ursprünglich gebildete Produkt erneut ein- oder mehrmal(s) besprüht wird, ist u.a. auch davon abhängig, welche Restfeuchte das gewünschte Produkt haben soll. Es versteht sich von selbst, daß ein Produkt mit besonders niedriger Restfeuchte nach dem letzten Besprühen eine längere Verweilzeit im Wirbelbett erforderlich macht als eines mit höherer.

Je nach Wunsch können durch die verschiedenen Düsen unterschiedliche Zusammensetzungen auf die bereits gebildeten Partikeloberflächen aufgebracht werden, so daß Partikel mit einem schichtförmigen Aufbau erhalten werden können. Es kann aber auch dazu dienen, eine gleichmäßigere Komverteilung zu erzielen.

Weiterhin kann die Anlage nicht nur mit Luft als Trägermedium betrieben werden. Es ist auch möglich, die gesamte Anlage im Kreislauf mit einem Inertgas, wie z.B. Stickstoff oder Kohlendioxid, zu fahren.

Die Anlage ist so ausgestaltet, daß die Parameter Flüssigkeitsmenge, Sprühdruck, rückgeführte Pulver- und Granulatmenge, Heißgasmenge, Heißgastemperatur, Warmluftmenge, Warmlufttemperatur usw. individuell regelbar sind. Daher lassen sich die zurückgeführte Pulver- und Granulatmenge, die eingespeiste Flüssigkeitsmenge und der Sprühdruck gezielt einstellen je nach gewünschten Eigenschaften hinsichtlich der Feuchte, der Korngröße und der Korngrößenverteilung des Endproduktes. Je nach Wunsch können in der beschriebenen Anlage Produkte hergestellt werden mit Partikelgrößen zwischen 50 und 1000µm. Je nach Fahrweise und gewählten Verfahrensparametern können die Partikel einen schichtförmigen (Zwiebelstruktur) Aufbau oder eine Agglomeratstruktur aufweisen.

Besonders gesteuert werden kann die Bildung der Partikel durch eine in die Anlage integrierte Sprühdüse, welche geeignet ist, um sprühgetrocknete Granulate herzustellen. Bei dieser Sprühdüse handelt es sich um ein Sprühsystem (B), die aus einer mit Heißwasser beheizbaren Zweistoff-Sprühdüse [(1), (2), (3)] besteht, welche wiederum mit einer um die Zweistoff-Sprühdüse angeordneten Pulver- und Granulatrückführung (4) und einer Heißgasumströmung (5) ausgestattet ist. Im Spezialfall kann die Pulver- und Granulatrückführung (4) koaxial um die Zweistoff-Sprühdüse angeordnet sein.

Vorteil dieses Sprühsystems ist, dass rückgeführter Feststoff unmittelbar am Austritt der Zweistoff-Sprühdüse mit den über die Zerstäubungsluft erzeugten Flüssigkeitströpfchen in Kontakt kommt. Damit die Partikel nicht verkleben und die Oberflächenfeuchte abgeführt werden kann, ist die Sprühdüse und die Pulver- und Granulatrückführung in einem Heißgasstrom eingeschlossen. Die Nachtrocknung auf die gewünschte Restfeuchte findet im Fließbett statt.

Insbesondere auch durch den Einbau dieses Sprühtrocknungssystems ist es möglich, Partikelgrößen gezielt herzustellen.

Ein besonderer Vorteil dieses Sprühtrocknungsverfahrens besteht daher darin, dass sich in einer einzigen Anlage ohne weitere Verfahrensschritte zur Nachbehandlung des Produkts in Abhängigkeit von den eingestellten Verfahrensparametern und von den verwendeten zu versprühenden flüssigen Medien δ-(D)-Mannit mit sehr unterschiedlichen Eigenschaften hinsichtlich Feuchte, der Korngröße und der Korngrößenverteilung im Pulver herstellen lässt.

Um besonders gute DC-Eigenschaften (DC = direkt tablettierbar) des sprühgetrockneten Stoffes zu erhalten, hier Mannit, ist es vorteilhaft, in der Sprühtrocknung gebildete Einzelpartikel zu agglomerieren. Zu diesem Zweck befindet sich senkrecht über dem Wirbelbett ein Sprühturm (B).

Die heiße wäßrige Mannitlösung wird über eine oder mehrere Zweistoffdüse(n) (5) (6), die mit Heißwasser (7) beheizt wird (werden), versprüht. Der erzeugte Sprühstrahl wird von einer um diese Düse angeordneten MannitPulver- und Granulatrückführung aus (9) und Heißgas (4) umströmt. Der Feststoff kristallisiert in dem Sprühstrahl aus, bildet Agglomerate und wird vom Fließbett aufgefangen. Das Fließbett wird von heißer Luft aus den Lufteinführungskammern (1) durchströmt und fluidisiert. Der Boden des Fließbettes ist ein Conidurboden, der für den gezielten Transport des Feststoffes zum Austrag sorgt, und auch eine definierte Verweilzeit des Feststoffes im Fließbett ergibt. Durch die Betthöhe, Sprüh- und Rückführmenge kann die Verweilzeit des Produkts im Prozessor gesteuert werden. Der Feststoff wird über mehrere in Reihe geschalteten Lufteinführungskammern (1) transportiert und auf eine Restfeuchte <0,3% getrocknet. Der Trocknungsvorgang erfolgt über die Länge des Fließbettes in einem bestimmten Temperaturprofil, um eine Überhitzung des Produktes zu vermeiden.

Die mit Wasser beladene und staubhaltige Wirbelluft wird über Dynamikfilter (G) gereinigt und durch die Abluftkammern (2) abgeführt. Die Dynamikfilter werden mit Druckluftstößen regelmäßig abgereinigt. Der abgereinigte Staub bindet die Sprühnebel aus der Sprühzone und verhindert Anbackungen an den Wänden.

Der getrocknete Feststoff fällt über Doppelpendelklappen (F) oder andere Austragssysteme in ein Dosiersystem der Rückführung (D). Das ausgetragene Produkt kann gegebenenfalls über ein Klassiersystem weiter aufgearbeitet werden. Das entstehende Überkorn (- und Unterkorn) kann über die Pulverrückführung (9) im Ventilator (E) gemahlen und zusammen mit dem Unterkorn (staubförmiges Mannitpulver mit Korngrößen kleiner 75 µm, insbesondere kleiner 40 µm) in den Sprühtrockner rückgeführt werden.

Ein Teilstrom wird am Austrag als fertiges Produkt (8) ausgeschleust. Das Produkt kann über ein Sieb klassiert werden, wobei das Überkorn (Remanenz, bzw. grobkörnige Pulveranteil) über die Saugseite des Mahlventilators (9A) rückgeführt, gemahlen und wieder in den Prozeß zurückgegeben werden kann. Dadurch werden u. a. die Produktverluste minimiert.

Der Ventilator (E) der Sprühtrocknungseinheit dient sowohl als Fördermittel für zurückzuführendes Produkt (Feststoffaufgabe druckseitig (9B)) als auch als Zerkleinerungseinheit für rückgeführtes Pulvermaterial (Feststoffaufgabe saugseitig (9A)). Die beiden Feststoffteilströme werden z.B. über die Drehzahl der Zellradschleusen (10A, 10B) gesteuert. Zurückgeführtes Pulver- und Granulatmaterial der Rückführleitungen (9) wird, wie oben bereits beschrieben, durch die besondere Ausgestaltung der Sprühtrocknungsdüse mit den entsprechenden Medien Flüssigkeit (Mannitlösung) (5), Sprühluft (6) und Heißluft (4) zusammengeführt.

Dem Ventilator (E) wird aus der Produktaustragszone des Prozessors Zuluft zugeführt, wenn die Klappe (H) geöffnet und die Klappe (J) geschlossen ist. Auf diese Art und Weise wird gleichzeitig aus dem Produkt der Feinstaub (<15µm) entfernt (pneumatische Klassierung). Gleichzeitig hat die Entfernung dieses Feinstaubs den Effekt, dass bei Verwendung dieses von Feinstaub befreiten Produktes höhere Tablettenhärten erreicht werden können.

Bei dem Teilstrom 9B besteht nach der Zellradschleuse 10B die Option, das Überkorn (Remanenz) aus der Rückführung abzusieben, um das Verfahren besser steuern zu können. Dieses Überkorn (Remanenz) kann saugseitig dem Mahlventilator (E) oder einer anderen Zerkleinerungsmaschine zugegeben, gemahlen und wieder dem Prozeß zugeführt werden.

Wie oben schon angedeutet, lässt sich die Qualität der Agglomerate und damit des Produktes über die Anlagenparameter wie Konzentration, Sprühdruck, Temperatur, Sprühmenge, Pulver- und Granulatrückführungsmenge, Hauptluftmenge, Staubabsaugung, Betthöhe usw. steuern. Durch eine Reduzierung der Höhe der Sprühdüse [(B)→(C)] über dem Fließbett ist es möglich, die Kornstruktur von einem Agglomerat (Beerenstruktur) in ein Granulat (Zwiebelstruktur) zu überführen. Bei der tiefstmöglichen Anordnung der Düsen (Granulationsdüsen (C)) kann die Pulverrückführung (9) über den Einfüllstutzen (3) erfolgen. Um kontinuierlich ein direkt tablettierbares Produkt zu erhalten, müssen sowohl die Kornstrukturen, aber auch Modifikation, Korngrößenverteilung, Wassergehalt, Dichte, usw. überwacht werden. Es wurde gefunden, dass das beste tablettierbare Produkt erhalten wird, wenn Mannit in einer feinen Nadelstruktur auskristallisiert.

Verunreinigungen durch andere Mannit-Modifikationen im Mannitgranulat verschlechtern die Tablettiereigenschaften. Insbesondere wurde gefunden, dass vor allem zunehmende Anteile an α-Mannit sich negativ auf die Tablettierbarkeit, die erzielbaren Tablettenhärten und auf die Oberflächenbeschaffenheit der Tabletten auswirken. In Produkten, die nach dem erfindungsgemäßen Verfahren hergestellt worden sind, ist an sich kein α-Mannit nachweisbar, insbesondere im Mannit, welches einen Anteil an δ-Modifikation von mehr als 95 Gew.-%, insbesondere von mehr als 98 Gew.-% aufweist.

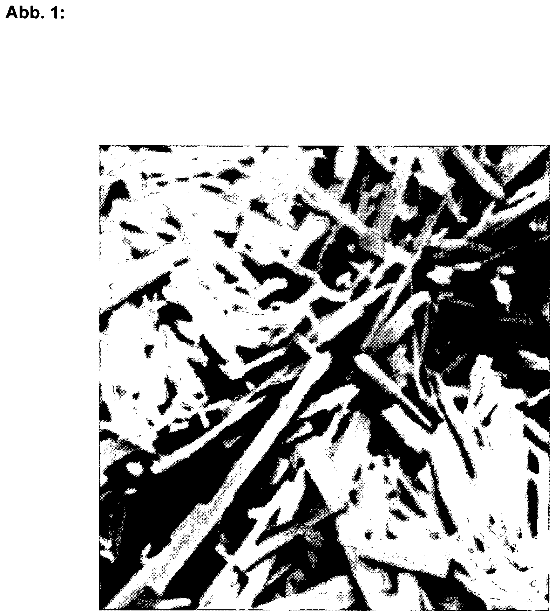

In **Abb.1** ist eine REM-Aufnahme eines Produkts mit einem Anteil an δ-Modifikation von mehr als 98% in einer 500-fachen Vergrößerung wiedergegeben.

Durch Versuche wurde gefunden, dass es notwendig ist, die eingestellten Parameter des Sprühtrocknungsprozesses einzuhalten und zu überwachen, um ein gleichbleibend, gut tablettierbares, reines δ-Mannit zu erhalten.

Erfindungsgemäß wird als Edukt D-Mannit mit einer Reinheit >98%, eingesetzt. Dieses Edukt wird als wässrige 40 - 50%-ge Lösung eingesetzt und in die Anlage mit einer Temperatur im Bereich von 60 bis 95 °C versprüht. Vorzugsweise wird die Lösung vor dem Versprühen auf eine Temperatur im Bereich von 70 bis 85 °C, insbesondere von 75 bis 80°C, erwärmt.

Erfindungsgemäß können an verschiedenen Stellen der Anlage Lösungen mit unterschiedlichen Mannitkonzentrationen eingesetzt werden. So hat es sich als sinnvoll erwiesen, Sprühdüsen oberhalb des Fließbetts in Richtung des Produktaustrags mit Lösungen mit höheren Mannitkonzentrationen zu beschicken als Sprühdüsen, welche sich am Anfang des Fließbetts befinden. Es kann daher zum Ende des Fließbetts hin eine Lösung mit einer Mannitkonzentration von etwa 60 Gew.-% bezogen auf die Gesamtlösung eingesetzt werden, wohingegen die Zweistoffdüse mit Pulverrückführung bevorzugt mit einer etwa 40 - 50%-gen wässrigen Lösung betrieben wird. Auf diese Weise können die Produkteigenschaften nochmals im gewünschten Sinn beeinflusst werden, wobei bei dieser Fahrweise die Anlagenparameter genau zu beachten sind.

Durch Variation der Parameter, Sprühdruck, Flüssigkeitsmenge, zurückgeführte Pulvermenge, Heißluftstrom und Temperatur der Heißluft lassen sich gezielt Partikelgrößen zwischen 50 bis 1000 µm einstellen.

Weiterhin wurde gefunden, dass die Parameter der erfindungsgemäß verwendeten Anlage folgendermaßen eingestellt werden müssen, um ein gleichbleibendes Produkt zu erhalten:
Der Sprühdruck der Zweistoffdüsen ist im Bereich von 2,5 bis 3,0 bar einzustellen.

Die Zufuhr von Heißgas am Kopf der Anlage kann den Prozess der Bildung von δ-Mannit unterstützen.

Die der Zweistoffdüse zugeführte Menge Heißgas ist so zu regulieren, dass etwa 1,5 bis 3 m³/(h kg Lösung) mit einer Temperatur von etwa 80 bis 110 °C gefördert werden. Es wurde gefunden, dass bei höherer Heißgaszufuhrmenge eine bessere Produktqualität erhalten wird, wenn mit niedrigerer Temperatur gearbeitet wird.

Die Rückführung des Feststoffes ist erfindungsgemäß so einzustellen, dass eine Rückführung im Bereich von 0,2 - 2,0 kg Feststoff/(h kg Lösung) erfolgt. Vorzugsweise wird im Bereich von 0,5 bis 1,5 kg Feststoff/(h kg Lösung) gearbeitet. Besonders günstig gestaltet sich der Prozess, wenn die Feststoffrückführung im Bereich von 0,5 bis 1,0 kg Feststoff/(h kg Lösung) liegt.

Zur Durchführung des Verfahrens muss in die Anlage vorgewärmte Luft eingespeist werden. Gute Ergebnisse werden erzielt, wenn die der Anlage zugeführte Luft auf eine Temperatur im Bereich von 45 - 120 °C vorgewärmt wird. Günstig ist es für den erfindungsgemäßen Prozess, wenn die Zuluft eine Temperatur im Bereich von 65 bis 110 °C aufweist. Besonders vorteilhaft für die Bildung eines δ-Mannitgranulates mit guten Tablettiereigenschaften ist es, wenn die Temperatur der eingespeisten Zuluft im Bereich von 70 bis 100 °C liegt.

Die zugeführte Zuluftmenge ist erfindungsgemäß so zu regeln, dass 1000 - 2000 m³/m² pro Stunde, insbesondere 1200 bis 1700 m³/m² pro Stunde, in die Anlage eingespeist werden.

In Verbindung mit den übrigen eingestellten Parametern liegen günstige Verfahrensbedingungen vor, wenn der Luftstrom in der Anlage so geführt wird, dass sich die Ablufttemperatur im Bereich von 30 -50 °C einstellt.

Weiterhin hat es sich als günstig herausgestellt, die Verfahrensbedingungen so zu regulieren, dass sich die im Wirbel- oder Fließbett befindliche Feststoffmenge auf eine Bettmenge von 50 - 150 kg/m² Bett einstellt. Besonders günstig ist es, wenn die Bettmenge im Bereich von 80 - 120 kg/m² Bett liegt.

Es wurde auch gefunden, dass sich der Prozess insbesondere durch gezielte Rückführung des Feststoffes mit ausgewählter Partikelgröße steuern lässt..

Bei Sprühversuchen hat es sich gezeigt, dass die Bildung von δ-Mannit aus β-Mannit nur erhalten wird, wenn die Zuluft des Mahlventilators staubfrei ist, d.h. die Klappe (H) ist geschlossen und die Klappe (J) offen, und die Zuluft kann zum Beispiel oberhalb der Prozessfilter direkt aus dem Prozessor gezogen werden, oder es wird temperierte und konditionierte Frischluft eingesetzt. Um den Sprühprozess stabil zu halten, muss mit Hilfe des Mahlventilators Feststoffgranulat aus dem Prozessor rückgeführt werden. Das Granulat wird sowohl vor als auch hinter dem Mahlventilator aufgegeben, durch die Einstellung der Drehzahlen der Zellradschleusen 10A und 10B kann das Verhältnis zwischen aufgemahlenem Pulver und ungemahlenem Granulat eingestellt werden. Wenn sich im Bett ein δ-Mannit-Gehalt von wesentlich über 50%, besser ein Gehalt von 60-70% gebildet hat, muss die Zuluft des Mahlventilators von staubfrei auf staubbeladen aus dem Prozessor umgestellt werden. In diesem Fall ist die Klappe (H) offen und die Klappe (J) geschlossen. So steigt der δ-Mannit-Gehalt weiter auf über 90%, bei längerer Prozessdauer; d. h. mit den eingestellten Parametern steigt der Gehalt sogar auf über 95% und erreicht sogar Werte von über 98%.

Wie aus dem Anlagenschema zu erkennen ist, kann eine Feststoffrückführung sowohl durch Feststoffentnahme aus dem Fließbett als auch durch Rückführung einer sehr feinteiligen Feststofffraktion, welche bei der Konfektionierung, d. h. Homogenisierung der Partikelgrösse durch Siebung und Abfüllung des hergestellten Produkts anfällt, erfolgen.

Es ist auch möglich, vor der Rückführung vorab Feststoff mit größeren Partikelquerschnitten im Ventilator (E) der Sprühtrocknungseinheit zu Pulver zu zerkleinern. Wie oben bereits angedeutet, kann der Pulverstrom durch die Einstellung der Drehzahl der Zellradschleusen (10A, 10B) gesteuert werden. Um vor der Rückführung rückzuführenden Feststoff auf die gewünschte Partikelgröße vorab zu vermahlen, ist dementsprechend die Drehzahl der Zellradschleuse 10A (B) so einzustellen, dass eine Rückführung über den Ventilator unter Vermahlung erfolgt.

Versuche haben gezeigt, dass das Gleichgewicht zur Bildung von δ-Mannit hin verschoben werden kann, wenn die durchschnittliche Partikelgröße des rückgeführten, im Ventilator (E) gemahlenen Pulvers kleiner als 75 µm ist. Insbesondere erfolgt bevorzugt die Bildung von δ-Mannit, wenn die durchschnittliche Teilchengröße des rückgeführten Pulvers geringer als 40 µm ist. Überraschend wurde gefunden, dass durch Rückführung eines Pulvers mit Teilchengrößen kleiner 20 µm Mannitpulver mit einem Anteil an δ-Fraktion von mehr als 90 % erhalten wird. Besonders überraschend wurde gefunden, dass insbesondere durch die Rückführung des sogenannten Staubanteils, der in der Produktaustragszone des Prozessors anfällt und üblicherweise aus dem Produkt entfernt wird, zu einem gleichmäßigen Produkt mit einem besonders hohen Anteil an δ-Fraktion führt. Die durchschnittliche Partikelgröße des Staubanteils liegt im Bereich von etwa 1 bis 20 µm, insbesondere im Bereich von 3 bis 15 µm. Außerdem wurde gefunden, dass der Staub aus der Rückführung zu einer stabilen Fahrweise in der Sprühzone des Prozessors führt.

Da durch Vermahlen im Ventilator (E) diese Partikelgrößen sich nur mit besonderem Aufwand erzielen lassen, wird insbesondere zu Beginn der Durchführung des Verfahrens bevorzugt der "staubförmige" Produktanteil aus der Pulverdosieranlage, der in der Anlage in der Leitung (9A) anfällt, durch Steuerung der Drehzahl der Zellradschleuse 10A durch Vermahlen in die Sprühtrocknung rückgeführt. Durch gleichzeitige Drosselung der Drehzahl der Zellradschleuse 10 B wird eine Rückführung grobkörnigen Mannitanteils reduziert.

Überraschenderweise wurde gefunden, dass im Prozess nach Einstellung des Gleichgewichts in Richtung der Bildung von δ-Mannit in einem Anteil von mehr als 90 % im Produkt der Prozess stabil weitergeführt werden kann, wenn ebenfalls das im Ventilator auf eine Partikelgröße von kleiner 75 µm vermahlene Pulver rückgeführt wird.

Auf diese Weise ist es überraschenderweise möglich, den Sprühtrocknungsprozess durch alleinige Rückführung des anfallenden "Pulveranteils" durch Regelung der Drehzahlen der Zellradschleusen 10A und 10B so einzustellen, dass die alleinige Bildung von δ-Mannit erfolgt. Anschließend kann auch der anfallende grobkörnigere Anteil (das sogenannte Überkorn) des gebildeten Mannitgranulats wieder in den Prozess rückgeführt werden, ohne eine Verschiebung des Gleichgewichts zu riskieren. Dieses hat den Vorteil, dass ein Anhaften besonders feinteiligen Sprühnebels an den Wänden der Anlage im Dauerbetrieb vermieden werden kann, und Störungen des Prozessablaufs unterbunden werden können.

Durch geeignete Wahl der Verfahrensparameter lässt sich also ein Produkt mit einem Gehalt an δ-Modifikation von mehr als 90 % herstellen. Durch ständige Kontrolle der hergestellten Produktqualität lässt sich der Anteil ohne weiteres auf einen Gehalt an δ-Modifikation auf über 95 % steigern.

Insbesondere bei optimalen Einstellungen der oben beschriebenen Anlagenparameter und Kontrolle der übrigen Verfahrensparameter wird im erfindungsgemäßen Verfahren als Produkt ein Mannit mit folgenden Eigenschaften erhalten:
- direkt tablettierbares Mannit
- Reinheit der δ-Modifikation > 98%
- Schüttdichte 350 - 500 g/l
- Restfeuchte < 0,3%
- Partikelverteilung: x₅₀ bei 200 µm: <10% <53µm + <15% >500µm
   x₅₀ bei 300µm: <10% <100µm + <10% >850µm
   x₅₀ bei 450µm: < 5% <100µm + <10% >850µm

Da die unterschiedlichen Modifikationen des Mannits sehr ähnlich sind, lassen sie sich aufgrund ihrer in der Analytik üblicherweise gemessenen Schmelztemperaturen in der DSC nicht unterscheiden. Die Identifizierung ist ausschließlich z.B. mittels X-Ray oder NIRS möglich.

Aufgrund der mit dem hergestellten Produkt erzielten Tablettenhärten lassen sich jedoch deutliche Unterschiede zu handelsüblichen Produkten feststellen. Im Vergleich zu einem handelsüblichen, kristallinem Produkt werden mit dem erfindungsgemäß hergestellten δ-Mannit Tabletten mit etwa 45 bis 70 % höheren Tablettenhärten erhalten.

**Tabelle 1: Tablettenhärten unter Verwendung von Mannit aus verschiedenen Herstellungsprozessen als Trägermaterial**

| | δ-Mannit Kristallisation | δ-Mannit Sprühgetrocknet | β-Mannit Sprühgetrocknet |
|---|---|---|---|
| Rieselfähigkeit | schlecht | gut | qut |
| Tablettenhärte (Preßkraft 20 kN) | Ca. 100 N | Ca. 260 N | Ca. 260 N |
| Tabletten härte Mit Binderzusatz (Preßkraft 20 kN) | Ca. 140 N | --- | --- |

Es hat sich gezeigt, dass das granulatförmige Mannit mit einem Gehalt an δ-Modifikation von mehr als 90 % in einfacher Weise dazu verwendet werden kann, einen pharmazeutischen Wirkstoff in Gegenwart von Feuchtigkeit oder eines geeigneten Lösungsmittels während der Umwandlung der Kristallstruktur aus der δ-Form in die β-Form fest an das Korn zu binden oder in das Granulatkorn einzubinden. Auf diese Weise lassen sich beispielsweise sehr gut pulverförmige Wirkstoffformulierungen herstellen, indem das erfindungsgemäße Produkt bei geeigneter Temperatur in einem Wirbelbett kontrolliert mit einer wirkstoffhaltigen Lösung oder Suspension besprüht wird. Während der Trocknung kann sich δ-Mannit in β-Mannit umwandeln, wobei der aufgesprühte Wirkstoff mit im Granulat und den das Granulat aufbauenden Kristallen eingeschlossen wird.

Zum besseren Verständnis und zur Verdeutlichung der Erfindung werden im folgenden ein allgemeines Fließschema des Aufbaus (Abb. 2) der beschriebenen Sprühtrocknungsanlage und Beispiele gegeben, die im Rahmen des Schutzbereichs der vorliegenden Erfindung liegen, nicht jedoch geeignet sind, die Erfindung auf diese Beispiele zu beschränken.

**Abb. 2** zeigt ein verallgemeinertes Fließschema einer möglichen Ausführungsform einer zur Durchführung des Verfahrens eingesetzten Sprühtrocknungsanlage, in der die gegebenen Ziffern und Buchstaben die folgenden Bedeutungen haben:
- 1: Lufteinführungskammern
- 2: Heizvorrichtungen
- 3: Einfüllstutzen
- 4: Heißluftzufuhr
- 5: Flüssigkeitszufuhr
- 6: Sprühluft
- 7: Heizmedium
- 8: Produkt
- 9: Pulver (9A feinteiliges Pulver (Staub), 9B grobkörniges Granulat)
- 10: Zellradschleuse (10A und 10B) zur Regelung der Pulverrückführung
- A: Fließbettapparat
- B: Sprühtrocknungseinheit
- C: Granulationsdüsen
- D: Feststoffdosiergerät
- E: Ventilator zur Pulver- und Granulatrückführung
- F: Schleusenklappen
- G: Dynamikfilter
- H: Zuluftklappe für staubhaltiges Gas
- J: Zuluftklappe für staubfreies Gas

Anhand der in der Beschreibung genannten und den in dem Fließschema gegebenen Komponenten ist es dem Fachmann ohne weiteres möglich, zur Durchführung des Verfahrens durch Auswahl im Handel erhältlicher Einzelkomponenten eine entsprechende Anlage zu erstellen. Es versteht sich für den auf dem Fachgebiet tätigen Fachmann von selbst, dass zum Betrieb der Anlage sowohl zusätzliche elektrische als auch mechanische Regelungseinheiten eingebaut werden müssen, um die Parameter im erfindungsgemäßen Verfahren, wie beschrieben, regeln und variieren zu können.

Zur Verdeutlichung der vorliegenden Erfindung sind der Beschreibung die folgenden Abbildungen angefügt:
**Abb.1****:** REM-Aufnahme eines Produkts mit einem Anteil an δ-Modifikation von mehr als 98% in einer 500-fachen Vergrößerung.
**Abb. 2****:** Verallgemeinertes Fließschema einer möglichen Ausführungsform einer zur Durchführung des Verfahrens eingesetzten Sprühtrocknungsanlage
**Abb. 3****:** Auftragung der gegen den Gehalt an δ-Mannit

Weiterhin sind zum besseren Verständnis, sofern Unklarheiten bestehen, auch die in der Beschreibung zitierten Literaturstellen und Patentschriften heranzuziehen, die hiermit als Teil der Offenbarung in die Beschreibung der vorliegenden Erfindung einbezogen werden.

Die in den Beispielen und der Beschreibung sowie in den Ansprüchen gegebenen Temperaturen gelten immer in °C. Wo nicht anders bezeichnet, sind Gehaltsangaben als Gew.-% bzw. Gewichtsverhältnisse aufgeführt.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew.- bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten %-Angaben als Gew.-%, mit Ausnahme von Verhältnissen, die in Volumenangaben wiedergegeben sind.

Die folgenden Beispiele zur Herstellung von verschiedenen DC δ-Mannit - Qualitäten dienen zur näheren Erläuterung der vorliegenden Erfindung.

### Beispiel 1

### Herstellung eines DC δ-Mannits mit einem mittleren Korn X₅₀ = 200 µm

Die Sprühtrocknungsanlage wird zur Vorbereitung mit ca. 70 kg/m² δ- Mannit als Bett befüllt. (Dieses vorgelegte Bett sollte möglichst die gewünschten Produkteigenschaften haben. Falls das zur Verfügung stehende Bettmaterial andere Eigenschaften besitzen sollte, so muss man die Anlage schonend anfahren, bis sich das Gleichgewicht in die gewünschte Richtung verschoben hat.)

Als Wirbel- und Zuluft wird die Anlage mit 1200 m³/m² h bei einer Temperatur von ca. 70° C betrieben. (Vor dem Anfahren der Anlage ist darauf zu achten, dass ausreichend Staub in der Anlage vorhanden ist. Es kann über die Pulverdosierung (D) Staub erzeugt und in die Anlage geblasen werden, indem gebildetes Produkt aus der saugseitigen Rückführung (9A) und der Dosierung (10 A) über den mit dem Ventilator (E) geführt und vermahlen wird.) Die Zuluft des Ventilators (E) wird über die Klappe (H) eingeführt. Ist genug Staub in der Anlage, kann die Dosierung (10 A) der Rückführung reduziert werden, und es wird mit dem Versprühen von Mannitlösung begonnen. Die versprühte Lösung hat eine Konzentration von ca. 40% und eine Temperatur von ca. 75° C. Bei einem Sprühdruck von ca. 3 bar (Sprühmedium ist Luft.) werden ca. 45 kg/m² h Lösung in der Anlage versprüht. Über die Pulverdosierung (D) werden ca. 0,5 kg Feststoff/(h kg Lösung) über die Rückführung (9, 10) in die Sprühzone rückgeführt. Die Zellräder (10 A, 10 B) werden so geregelt, dass immer eine ausreichende Menge Produkt (9A, 10A) im Ventilator (E) gemahlen und mit dem ungemahlenen Produkt (9B, 10B) wieder in die Anlage gefördert wird.

In der Anlage bildet sich durch die Verdunstung des Wassers ein Gleichgewicht mit einer Betttemperatur von ca. 35-40° C. Die Ablufttemperatur beträgt etwa 30-33° C. (Es ist darauf zu achten, dass die Abluft möglichst gesättigt ist (ca. 80 90% rel. Feuchte). Dies ist von Vorteil für den Wirkungsgrad des Prozesses als auch für den Kristallisationsvorgang des Mannits.) Auf diese Art und Weise erhält man die beste Kristallstruktur und die reinste δ-Modifikation des Mannits. Da der Ventilator (E) seine Zuluft aus der Produktaustragszone vor den Schleusenklappen (F) der Anlage (A) holt, und somit das ausgetragene Produkt durch die pneumatische Klassierung staubarm ist, erhält man am Produktaustrag (8) das δ-Mannit mit hervorragenden Eigenschaften für die direkte Tablettierung. Um DC δ-Mannit mit der gewünschten Korngrößenverteilung zu erhalten, kann es nach der Austragsschleuse (F), d. h. vor dem Produktaustrag (8) und der Pulverdosierung (D), gesiebt werden. Es ist für den Prozess von Vorteil, auch das Überkorn aus dem rückzuführenden Produkt (9 B, 10B) auszusieben, da dieses sich sonst in der Sprühzone weiter anreichert und im Fließbett Probleme bereiten kann. Das ausgesiebte Unter- und Überkorn kann saugseitig dem Ventilator (E) zugeführt, gemahlen werden, und zusammen mit den anderen rückgeführten Feststoffteilströmen (9A, 10 A, 9B, 10 B) wieder dem Prozess zugeführt werden. Auf diese Art und Weise werden die Produktverluste minimiert, und der Prozess läuft durch die zusätzliche Staubrückführung (gemahlenes Produkt) stabiler.

### Beispiel 2

### Herstellung eines DC δ-Mannits mit einem mittleren Korn X₅₀ = 300 µm

Wie in Beispiel 1 beschrieben wird die Sprühtrocknungsanlage zur Vorbereitung mit ca. 100 kg/m² δ-Mannit als Bett befüllt und angefahren.

Als Wirbel- und Zuluft wird die Anlage mit 1500 m³/m² h bei einer Temperatur von ca. 85° C betrieben. Die zu versprühende Mannit-Lösung hat eine Konzentration von ca. 45% bei einer Temperatur von ca. 80° C. Bei einem Sprühdruck von ca. 3 bar (Sprühmedium ist Luft) wird eine Lösungsmenge von ca. 65 kg/m² h in der Anlage versprüht. Die Zellräder (10 A, 10 B) werden so geregelt, dass immer eine ausreichende Menge Produkt (9A, 10A) im Ventilator (E) gemahlen und mit dem ungemahlenen Produkt (9B, 10B) wieder in die Anlage gefördert wird. Die Zuluft des Ventilators (E) wird über die Klappe (H) eingeführt.

In der Anlage bildet sich durch die Verdunstung des Wassers ein Gleichgewicht mit einer Bettemperatur von ca. 40° C. Die Ablufttemperatur beträgt ca. 35-40° C. Es ist darauf zu achten, dass die Abluft möglichst gesättigt ist (ca. 80 - 90% rel. Feuchte). Das Überkorn aus dem rückzuführenden Produkt (9B,10B) wird ausgesiebt, da dieses sich sonst in der Sprühzone weiter anreichert und im Fließbett Probleme bereitet. Das ausgesiebte Unter- und Überkorn wird saugseitig dem Ventilator (E) zugeführt und gemahlen. Es wird zusammen mit den anderen rückgeführten Feststoffteilströmen (9 A, 10 A, 9 B, 10 B) wieder dem Prozess zugeführt.

### Beispiel 3

### Herstellung eines DC δ-Mannits mit einem mittleren Korn X₅₀ = 450 µm

Wie in Beispiel 1 beschrieben, wird die Sprühtrocknungsanlage zur Vorbereitung mit ca. 120 kg/m² δ-Mannit als Bett befüllt. Als Wirbel- und Zuluft wird die Anlage mit 1700 m³/m² h bei einer Temperatur von ca. 100° C betrieben.

Das Heißgas wird in einer Größenordnung von ca. 1,6 m³/(h kg Lösung) bei einer Temperatur von ca. 80° C der Sprühzone zugeführt. Sind alle diese Parameter eingestellt, kann mit dem Versprühen von Mannitlösung begonnen werden.

Die Lösung hat eine Konzentration von ca. 50% bei einer Temperatur von ca. 90° C. Bei einem Sprühdruck von ca. 3,5 bar (Sprühmedium ist Luft.) wird eine Lösungsmenge von ca. 100 kg/m² h in der Anlage versprüht. Über die Pulverdosierung (D) wird über die Rückführung (9, 10) Bett/Produkt von ca. 0,8 - 1,0 kg Feststoff/(h kg Lösung) in die Sprühzone rückgeführt. Die Zellräder (10A, 10B) werden so geregelt, so dass immer eine ausreichende Menge Produkt (9A, 10A) im Ventilator (E) gemahlen und mit dem ungemahlenen Produkt (9B, 10 B) wieder in die Anlage gefördert wird.

In der Anlage bildet sich durch die Verdunstung des Wassers ein Gleichgewicht mit einer Bettemperatur von ca. 40-45° C. Die Ablufttemperatur beträgt ca. 40° C. Es ist darauf zu achten, dass die Abluft möglichst gesättigt ist (ca. 80 - 90% rel. Feuchte).

Das Überkorn aus dem rückzuführenden Produkt (9 B, 10B) wird ausgesiebt, da dieses sich sonst in der Sprühzone anreichert und im Fließbett Probleme bereitet. Das ausgesiebte Unter- und Überkorn wird saugseitig dem Ventilator (E) zugeführt und gemahlen. Die Zuluft des Ventilators (E) wird über die Klappe (H) zugeführt. Es wird zusammen mit den anderen rückgeführten Feststoffteilströmen (9A, 10 A / 9B, 10 B) wieder dem Prozess zugegeben.

## Patentansprüche

1. Verfahren zur Herstellung von direkt tablettierbarem Mannit mit einem Gehalt an δ-Modifikation von mehr als 90 %, **dadurch gekennzeichnet, dass**
a) ein Wirbelbett aus sehr feinteiligem β-Mannitpulver hergestellt wird, indem DC-β-Mannit als Bett im Prozessor vorgelegt wird und
b) in einem ersten Schritt als Edukt eine wässrige D-Mannit-Losung, Sprühgas, das β-Mannitpulver und Heißluft zusammengeführt werden, und
c) das entstehende granulatförmige Produkt in das Wirbel- oder Fließbett fällt, aufgenommen und fluidisiert wird, gegebenenfalls in einem oder mehreren Granulierungsschritt(en) mit weiterem flüssigen Medium besprüht, getrocknet, und im Fließbett in Richtung der Dosieranlage weitertransportiert wird,
und anschließend
d) eine Teilmenge ungemahlen als pulver- und/oder granulatförmiges Material in den Prozess zurückgeführt wird,
wobei
e) im Anfahrprozess die Zuluft des Mahlventilators (E) staubfrei ist, in der Anlage die Klappe (H) geschlossen ist und die Klappe (J) geöffnet ist und die Luft aus der Abluft des Prozessors nach den Filtern (G) entnommen wird und
f) im Prozessor eine Temperatur von max. ca. 35 - 45 °C sowie ein Sprühdruck von max. 2,5 - 3,0 bar eingestellt werden, wobei
die D-Mannit-Lösung hergestellt ist aus D-Mannit mit einem Mannitgehalt von >98 Gew.%,
und die Rückführung ungemahlenen Pulvers in einer Menge von ca. der 3-fachen Menge erfolgt, bezogen auf die aus dem Prozess entnommene Produktmenge,
und das rückgeführte Pulver in der Anlage den Weg 9B/10B nimmt, wobei der Weg 9A/10A geschlossen ist, wodurch die Bildung von δ-Mannit einsetzt und die Konzentration an δ-Mannit unter den eingestellten Bedingungen kontinuierlich zunimmt,
und
g) wenn der Gehalt an δ-Mannit im gebildeten Produkt auf etwa 60 - 70 Gew.-% gestiegen ist, die Rückführung von staubhaltiger Zuluft umgestellt wird, indem in der Anlage die Klappe (H) geöffnet und die Klappe (J) geschlossen werden, wodurch der Gehalt an δ-Mannit auf über 90 Gew.-% im Produkt gesteigert wird.

2. Verfahren gemäß Anspruch 1, **dadurch gekennzeichnet, dass** nach der Einstellung des Gleichgewichts unvermahlenes δ-Mannit-Granulat rückgeführt wird.

3. Verfahren gemäß Anspruch 1 oder 2, **dadurch gekennzeichnet, dass** eine wässrige 40 - 50%-ige D-Mannit-Lösung (bezogen auf das Gewicht der Gesamtlösung) als Edukt eingesetzt wird und mit einer Temperatur im Bereich von 60 bis 95 °C versprüht wird.

4. Verfahren gemäß der Ansprüche 1 bis 3, **dadurch gekennzeichnet, dass** sowohl als Sprühgas als auch als Träger- und Heizgas Luft oder ein Inertgas ausgewählt aus der Gruppe N₂ und CO₂ verwendet wird.

5. Verfahren gemäß der Ansprüche 1 bis 4, **dadurch gekennzeichnet, dass** das Gas im Kreislauf geführt wird und das im Kreislauf geführte Gas durch Filter von Partikeln befreit wird, im Kondensator getrocknet, gegebenenfalls aufgeheizt, und erneut den Sprühdüsen zugeführt und in das Fließbett eingeführt wird.

6. Verfahren gemäß der Ansprüche 1 bis 5, **dadurch gekennzeichnet, dass** durch Variation der Parameter, Sprühdruck, Sprühmenge, Mannitkonzentration, zurückgeführte Pulver- oder Granulatmenge, Heißluftstrom und Temperatur der Heißluft gezielt Partikelgrößen zwischen 50 bis 1000 µm hergestellt werden.

7. Verfahren gemäß Anspruch 6, **dadurch gekennzeichnet, dass** die der Anlage zugeführte Luft auf eine Temperatur im Bereich von 45 - 120 °C vorgewärmt wird und die Zuluft in einer Menge im Bereich von 1000 - 2000 m³/m² pro Stunde zugeführt wird, wodurch sich die Ablufttemperatur im Bereich von 30 - 50 °C einstellt.

8. Verfahren gemäß Anspruch 6, **dadurch gekennzeichnet, dass** die Feststoffrückführung in einer Menge im Bereich von 0,2 - 2,0 kg Feststoff/(h kg Lösung) erfolgt.

9. Verfahren gemäß Anspruch 6, **dadurch gekennzeichnet, dass** durch Einstellung der Parameter Sprühdruck, Flüssigkeitsmenge, Mannitkonzentration, zurückgeführte Feststoffmenge, Heißluftstrom und Temperatur der Heißluft die im Wirbel- oder Fließbett befindliche Pulvermenge auf eine Bettmenge im Bereich von 50 - 150 kg/m² Bett eingestellt wird.

## Claims

1. Process for the preparation of directly compressible mannitol having a content of the δ modification of greater than 90%, **characterised in that**
a) a fluidised bed comprising very finely divided β-mannitol powder is produced by placing DC β-mannitol as bed in the processor and
b) in a first step, as starting material, an aqueous D-mannitol solution, spray gas, the β-mannitol powder and hot air are combined, and
c) the granular product formed falls into the fluidised bed, is taken up and fluidised,
optionally, in one or more granulation step(s), sprayed with further liquid medium, dried and and transported further in the fluidised bed in the direction of the metering unit,
and subsequently
d) some of the unground material in powder and/or granular form is recycled into the process,
where
e) in the start-up process, the feed air to the grinding fan (E) is dust-free, flap (H) in the plant is closed and flap (J) is open, and the air from the processor exhaust is withdrawn after the filters (G)
and
f) a temperature of max. about 35 - 45°C and a spray pressure of max. 2.5 - 3.0 bar are set in the processor,
where
the D-mannitol solution is prepared from D-mannitol having a mannitol content of >98% by weight,
and the recycling of unground powder takes place in an amount of about 3 times the amount, based on the amount of product withdrawn from the process,
and the recycled powder takes path 9B/10B in the plant, with path 9A/10A closed, causing the formation of δ-mannitol to commence and the concentration of δ-mannitol to increase continuously under the conditions set,
and
g) when the content of δ-mannitol in the product formed has risen to about 60 - 70% by weight, the recycling of dust -containing feed air is switched over by opening flap (H) and closing flap (J) in the plant, causing the content of δ-mannitol to rise to greater than 90% by weight in the product.

2. Process according to Claim 1, **characterised in that**, after establishment of the equilibrium, unground δ-mannitol granular material is recycled.

3. Process according to Claim 1 or 2, **characterised in that** an aqueous 40 - 50% D-mannitol solution (based on the weight of the entire solution) is employed as starting material and is sprayed with a temperature in the range from 60 to 95°C.

4. Process according to Claims 1 to 3, **characterised in that** air or an inert gas selected from the group N₂ and CO₂ is used both as spray gas and as carrier gas and heating gas.

5. Process according to Claims 1 to 4, **characterised in that** the gas is circulated, and the circulated gas is freed from particles by filters, dried in the condenser, heated if necessary, and fed to the spray nozzles again and introduced into the fluidised bed.

6. Process according to Claims 1 to 5, **characterised in that** particle sizes between 50 to 1000 µm are produced specifically by variation of the parameters spray pressure, spray amount, mannitol concentration, recycled amount of powder or granular material, hot-air flow rate and hot-air temperature.

7. Process according to Claim 6, **characterised in that** the air fed to the plant is pre-warmed to a temperature in the range 45 - 120°C, and the feed air is fed in an amount in the range 1000 - 2000 m³/m² per hour, giving a exhaust-air temperature in the range 30 - 50°C.

8. Process according to Claim 6, **characterised in that** the solids recycling is carried out in an amount in the range 0.2 - 2.0 kg of solid/(h kg of solution).

9. Process according to Claim 6, **characterised in that** the amount of powder present in the fluidised bed is set to an amount of bed in the range 50 - 150 kg/m² of bed by adjustment of the parameters spray pressure, amount of liquid, mannitol concentration, recycled amount of solid, hot-air flow rate and hot-air temperature.

## Revendications

1. Procédé pour la préparation de mannitol directement compressible présentant une teneur en la modification δ supérieure à 90 %, **caractérisé en ce que**
a) un lit fluidisé qui comprend de la poudre de β-mannitol très finement divisée est constitué en plaçant du DC β-mannitol en tant que lit dans le dispositif de traitement et
b) au niveau d'une première étape, en tant que matériau de départ, une solution aqueuse de D-mannitol, un gaz de pulvérisation, la poudre de β-mannitol et de l'air chaud sont combinés, et
c) le produit granulaire qui est formé tombe dans le lit fluidisé, est absorbé et fluidisé,
en option, au niveau d'une ou de plusieurs étape(s) de granulation, il est pulvérisé à l'aide d'un autre milieu liquide, séché et transporté encore dans le lit fluidisé dans la direction de l'unité de mesure,
et subséquemment
d) une certaine part du matériau non broyé sous forme pulvérulente et/ou granulaire est recyclée dans le processus,
où
e) au niveau du processus de démarrage, l'air qui est alimenté sur le ventilateur de broyage (E) est exempt de poussière, un volet (H) dans l'installation est fermé et un volet (J) est ouvert, et l'air qui provient de rejets du dispositif de traitement est extrait après les filtres (G),
et
f) une température maximum d'environ 35 - 45°C et une pression de pulvérisation maximum de 2,5 - 3,0 bars sont instaurées à l'intérieur du dispositif de traitement,
où
la solution de D-mannitol est préparée à partir de D-mannitol qui présente une teneur en mannitol > 98% en poids,
et le recyclage de la poudre non broyée est réalisé selon une quantité qui est égale à environ 3 fois la quantité, sur la base de la quantité de produit extraite à partir du processus,
et la poudre recyclée chemine selon la voie 9B/10B dans l'installation, la voie 9A/10A étant fermée, ce qui provoque le début de la formation de δ-mannitol et l'augmentation de la concentration en δ-mannitol en continu sous les conditions définies,
et
g) lorsque la teneur en δ-mannitol dans le produit qui est formé a crû jusqu'à environ 60 - 70% en poids, la commutation sur le recyclage de l'air d'alimentation contenant de la poussière est réalisée en ouvrant le volet (H) et en fermant le volet (J) dans l'installation, ce qui provoque l'augmentation de la teneur en δ-mannitol jusqu'à plus de 90 % en poids dans le produit.

2. Procédé selon la revendication 1, **caractérisé en ce que**, après l'établissement de l'équilibre, le matériau granulaire δ-mannitol non broyé est recyclé.

3. Procédé selon la revendication 1 ou 2, **caractérisé en ce qu'**une solution aqueuse à 40 - 50 % de D-mannitol (sur la base du poids de la solution complète) est utilisée en tant que matériau de départ et est pulvérisée à une température qui s'inscrit à l'intérieur de la plage qui va de 60 à 95 °C.

4. Procédé selon les revendications 1 à 3, **caractérisé en ce que** de l'air ou un gaz inerte qui est sélectionné au sein du groupe qui est constitué par N₂ et CO₂ est utilisé à la fois en tant que gaz de pulvérisation et en tant que gaz porteur et gaz de chauffage.

5. Procédé selon les revendications 1 à 4, **caractérisé en ce qu'**une circulation du gaz est réalisée, et le gaz qui circule est libéré de particules par des filtres, est séché dans le condenseur, est chauffé si nécessaire, est alimenté sur les éjecteurs de pulvérisation à nouveau et est introduit à l'intérieur du lit fluidisé.

6. Procédé selon les revendications 1 à 5, **caractérisé en ce que** des particules présentant des tailles entre 50 et 1000 µm sont produites de façon spécifique en faisant varier les paramètres que sont la pression de pulvérisation, la quantité de pulvérisation, la concentration en mannitol, la quantité recyclée de matériau pulvérulent ou granulaire, le débit d'écoulement de l'air chaud et la température de l'air chaud.

7. Procédé selon la revendication 6, **caractérisé en ce que** l'air qui est alimenté sur l'installation est préchauffé jusqu'à une température qui s'inscrit à l'intérieur de la plage de 45 - 120°C, et l'air d'alimentation est alimenté selon une quantité qui s'inscrit à l'intérieur de la plage de 1000 - 2000 m³/m² par heure, ce qui donne une température d'air de rejet qui s'inscrit à l'intérieur de la plage de 30 - 50°C.

8. Procédé selon la revendication 6, **caractérisé en ce que** le recyclage des solides est mis en œuvre selon une quantité qui s'inscrit à l'intérieur de la plage de 0,2 - 2,0 kg de solides/(h kg de solution).

9. Procédé selon la revendication 6, **caractérisé en ce que** la quantité de poudre qui est présente dans le lit fluidisé est établie à une quantité de lit qui s'inscrit à l'intérieur de la plage de 50 - 150 kg/m² de lit au moyen d'un réglage des paramètres que sont la pression de pulvérisation, la quantité de liquide, la concentration en mannitol, la quantité recyclée de solides, le débit d'écoulement de l'air chaud et la température de l'air chaud.
